# EUROPÄISCHE PATENTSCHRIFT

(11) **EP 1 213 009 B1**
(45) Veröffentlichungstag und Bekanntmachung des Hinweises auf die Patenterteilung: **25.10.2006**
(21) Anmeldenummer: 01120535.8
(22) Anmeldetag: 29.08.2001
(51) Int. Cl.: A61K 8/89, A61Q 5/08, A61Q 5/10

(54) **Pulverförmiges Mittel zum Blondieren oder Färben von Haaren und Verfahren zu dessen Herstellung**
Composition in powder form for bleaching or dyeing of hair and its preparation process
Composition en poudre pour blondir ou colorer les cheveux et son procédé de préparation

(30) Priorität: 05.12.2000 DE 10060467
(43) Veröffentlichungstag der Anmeldung: 12.06.2002
(73) Patentinhaber: Wella Aktiengesellschaft, 64274 Darmstadt (DE)
(72) Erfinder: Hess, Gabriele, 64390 Erzhausen (DE); Lauscher, Dirk, Dr., 64342 Seeheim-Jugenheim (DE); Schmenger, Jürgen, 64331 Weiterstadt (DE)

(56) Entgegenhaltungen:
- EP-A- 0 583 767
- EP-A- 0 630 643
- EP-A- 1 136 064
- WO-A-98/00104
- US-A- 5 654 362

## Beschreibung

Die vorliegende Erfindung beschreibt ein pulverförmiges Mittel zum Blondieren oder Färben von Haaren sowie ein Verfahren zu dessen Herstellung.

Herkömmliche pulverförmige Mittel, wie zum Beispiel Pulverhaarfarben, Blondiermittel und Trockenshampoos, sind entweder mit diversen Trocknungsmitteln oder Bindemitteln formuliert und/oder werden mit entsprechenden Entstaubungstechniken behandelt.

Aus dem Stand der Technik sind eine Vielzahl von Verfahren zur Herstellung von mehr oder weniger staubfreien Pulvern bekannt, wobei die Entstaubung der Pulver in der Regel mit Hilfe von flüssigen Ölen oder Wachsen, beispielsweise Silikonölen und Silikonwachsen oder Paraffinölen und Paraffinwachsen, erfolgt. Aus der EP-OS 0583767 ist ein Verfahren zur Herstellung von staubfreien Formulierungen bekannt, bei dem das Pulver mit einer inerten Flüssigkeit, beispielsweise Silikonölen oder natürlichen Ölen, behandelt wird. In der EP-PS 0560088 wird die Entsstaubung von pulverförmigen Blondiermitteln mit Ölen oder flüssigen Wachsen, beispielsweise Silikonölen oder Paraffinölen, beschrieben. In der DE-OS 19600225 wird die Entstaubung von pulverförmigen Haarfärbemitteln mit Ölen oder flüssigen Wachsen, beispielsweise Silikonölen, Jojobaöl oder bestimmten Fettsäuren und Fettalkoholen, beschrieben. Aus der DE-OS 19853653 ist es bekannt, pulverförmige Mittel mit bestimmten, bei 25 °C flüssigen Lösemitteln, zum Beispiel Kohlenwasserstoffen, Alkoholen, Estern und Ketonen, zu entstauben. Die EP-OS 0630643 beschreibt ein Verfahren zur Herstellung pulverförmiger Mittel zum Färben und Blondieren von Haaren, bei dem höherschmelzende Wachse aufgeschmolzen werden und sodann in flüssiger Form auf das Pulver aufgesprüht werden. Aus der WO 98/00104 sind Silikonelastomere enthaltende pulverförmige Hautbehandlungsmittel bekannt. Die vorstehend beschriebenen Verfahren sind jedoch nicht in jeder Hinsicht optimal, so dass die nach diesen Verfahren erhaltenen pulverförmigen Mittel weiterhin einige Nachteile, insbesondere im Hinblick auf die Lagerstabilität, aufweisen.

Bei der Herstellung von pulverförmigen Zubereitungen ist neben der Entstaubung und Trockenhaltung des Mittels die Stabilisierung der pulverförmigen Bestandteile während der Lagerung (d.h. während der Aufbewahrung bis zum Einsatz) von entscheidender Bedeutung. Um eine derartige Stabilisierung der Pulver während der Lagerung zu erzielen, müssen die teilweise sehr reaktiven pulverförmigen Bestandteile der Pulvermischung so behandelt werden, dass sie weder chemisch abreagieren können noch Agglomerate bilden können.

Überraschenderweise wurde nunmehr gefunden, dass pulverförmige kosmetische Mittel mit einem Gehalt an bestimmten Silikonelastomeren die vorgenannten Nachteile nicht aufweisen.

Gegenstand der vorliegenden Erfindung ist daher ein pulverförmiges Blondiermittel auf der Basis einer festen Perverbindung oder ein pulverförmiges Haarfärbemittel auf der Basis oxidativer und/oder nicht-oxidativer Farbstoffe, welches dadurch gekennzeichnet ist, dass es mindestens ein Silikonelastomer enthält, das ausgewählt ist aus Polymeren, welche durch Vernetzung von (I) Si-H-Gruppen enthaltenden Polysiloxanen mit (IIa) einem alpha, omega-Dien oder (IIb) einem Vinylpolysiloxan in Gegenwart eines Katalysators und eines Lösungsmittels erhalten werden.

Als Silikonelastomere werden vernetzte Silikonpolymere verwendet, die beispielsweise durch Vernetzung von (I) Si-H-Gruppen enthaltenden Polysiloxanen mit (IIa) einem alpha, omega-Dien oder (IIb) einem Vinylpolysiloxan in Gegenwart eines geeigneten Katalysators (wie zum Beispiel eines Platinkatalysators) und eines geeigneten Lösungsmittels (beispielsweise eines niedrig-molekularen linearen oder zyklischen Polysiloxans) erhalten werden. Derartige Silikonelastomere sowie ein Verfahren zu deren Herstellung werden beispielsweise in der US-PS 5654362, auf die hiermit ausdrücklich Bezug genommen wird, beschrieben. Vorzugsweise werden Silikonelastomere mit einer molaren Masse von mehr als 1000 g/mol verwendet. Besonders bevorzugte Silikonelastomere sind das Dimethicone/ Vinyldimethicone Crosspolymer und das Cyclopentasiloxan (and) Dimethicone Crosspolymer, die beispielsweise unter der Handelsbezeichnung DOW CORNING^{®} 9506 und DOW CORNING^{®} 9040 von der Dow Corning Corp. vertrieben werden.

Die bevorzugte Einsatzmenge der Silikonelastomere beträgt etwa 0,01 bis etwa 50 Gewichtsprozent, insbesondere etwa 0,1 bis etwa 20 Gewichtsprozent, wobei eine Einsatzmenge von 1 bis 10 Gewichtsprozent besonders bevorzugt ist.

Das pulverförmige kosmetische Mittel kann alle für derartige Mittel üblichen und bekannten Inhaltsstoffe enthalten, beispielsweise Tenside und Emulgatoren aus der Gruppe der anionischen, nichtionischen oder ampholytischen oberflächenaktiven Verbindungen, beispielsweise Fettalkoholsulfate, Alkansulfonate, Olefinsulfonate, Fettalkoholpolyglykolethersulfate, Alkylpolyglykoside und ethoxylierte Fettalkohole, Fettsäuren, Alkylphenole, Sorbitanfettsäureester und Fettsäurealkanolamide; Verdicker und Gelbildner wie zum Beispiel Bentonite, Fettalkohole, Fettsäuren, Paraffinöle, Silikonöle, Fettsäureester, Methylcellulose oder Hydroxyethylcellulose, Stärke, synthetische Polymerisate wie Polyvinylpyrrolidon und Polyacrylate, oder Biopolymere wie Guar Gum, Xanthan Gum, Traganth, Alginsäure und Natriumalginat; Stabilisatoren für Peroxoverbindungen wie zum Beispiel Silikate; sowie Komplexbildner; Parfümöle, haarfestigende Polymere und Pflegestoffe wie kationische Polymere, Lanolinderivate, Jojobaöl, Octylstearat, Isoparaffine, Cholesterin, Pantothensäure, Proteinderivate und Proteinhydrolysate, Provitamine und Vitamine, Pflanzenextrakte und anorganische oder organische Salze, beispielsweise die Aluminium-, Natrium-, Kalium- und Magnesiumsalze höherer Fettsäuren (zum Beispiel Stearate, Palmitate, oleate, Laurate Tallate oder 12-Hydroxystearate), sowie Puffersubstanzen und Mittel zur Einstellung des pH-Wertes, beispielsweise organische und anorganische Säuren oder Basen sowie Alkali-, Erdalkali- oder Ammoniumsalze.

Diese Zusätze werden in dem erfindungsgemässen pulverförmigen kosmetischen Mittel in für derartige Mittel üblichen Mengen eingesetzt; beispielsweise die Tenside und Emulgatoren in einer Konzentrationen von 0,2 bis 30 Gewichtsprozent und die Verdickungsmittel in einer Konzentrationen von 0,1 bis 25 Gewichtsprozent (jeweils bezogen auf das gebrauchsfertige Mittel).

Handelt es sich bei dem pulverförmigen kosmetischen Mittel um ein Haarfärbemittel ("Färbepulver") so enthält es übliche oxidative Farbstoffe aus der Gruppe der sogenannten Entwicklersubstanzen und Kupplersubstanzen und/oder nicht-oxidative Farbstoffe aus der Gruppe der Triphenylmethanfarbstoffe, aromatischen Nitrofarbstoffe, Azofarbstofte, Chinonfarbstoffe und kationischen oder anionischen Farbstoffe. Diese Farbstoffe sind in dem Färbepulver in einer Gesamtmenge von etwa 0,1 bis 25 Gewichtsprozent enthalten.

Wenn es sich bei dem pulverförmigen kosmetischen Mittel um ein Mittel zum Entfärben, Bleichen oder Blondieren von Haaren ("Blondierpulver") handelt, so enthält dieses Pulver feste Perverbindungen wie zum Beispiel Persulfate, Percarbonate, Perborate oder Wasserstoffperoxidaddukte wie Harnstoffperoxid. Diese Perverbindungen werden in dem Blondierpulver in einer Menge von etwa 25 bis 70 Gewichtsprozent eingesetzt.

Die vorstehend angegeben Einsatzmengen beziehen sich jeweils auf die Gesamtmenge des erfindungsgemässen pulverförmigen kosmetischen Mittels.

Die Herstellung des erfindungsgemässen pulverförmigen kosmetischen Mittels erfolgt in der Regel durch einfaches Vermischen des die übrigen Bestandteile enthaltenden Pulvers oder der einzelnen Pulverbestandteile mit dem Silikonelastomeren in einem geeigneten Mischer bei Raumtemperatur (etwa 10 bis 35 °C), wobei das Silikonelastomere gegebenenfalls in einem natürlichen oder synthetischen Öl (beispielsweise bei 25 °C flüssigen Silikonölen oder Paraffinölen, Pflanzenölen etc.) dispergiert wird, um so eine feinere Verteilung zu ermöglichen. Als Mischer können prinzipiell alle bekannten und üblichen Mischer, beispielsweise Trommelmischer, Taumelmischer, Doppelkonusmischer, Schneckenmischer, Pflugscharmischer, Fliessbettmischer, Telex-Mischer, Drais-Kneter oder pneumatische Mischer, verwendet werden, während die Dispergierung des Silikonelastomeren in dem Öl vorzugsweise mittels eines Homogenisators erfolgt.

Das erfindungsgemässe pulverförmige kosmetische Mittel ist nicht nur völlig staubfrei, gut rieselfähig und problemlos mit Wasser oder wässrigen Zubereitungen vermischbar, sondern ist zudem sehr leicht herstellbar und weist aufgrund des Gehaltes an Silikonelastomeren eine hervorragende Lagerstabilität auf.

Ein weiterer Gegenstand der vorliegenden Anmeldung ist daher die Verwendung der vorgenannten Silikonelastomeren zur Herstellung von staubfreien, lagerstabilen pulverförmigen kosmetischen Mitteln.

Die nachfolgenden Beispiele sollen die Erfindung näher erläutern ohne sich hierbei auf diese zu beschränken.

### Beispiele

### Beispiel 1: Blondierpulver staubfrei (Anwendung mit konventioneller Wasserstoffperoxid Mischung)

| | |
|---|---|
| 11,6 g | Magnesiumperoxid |
| 37,8 g | Ammoniumperoxodisulfat |
| 11,3 g | Magnesiumcitrat |
| 26,3 g | Magnesiumoxid |
| 3,0 g | Xanthan Gum |
| 5,0 g | Dimethicone/Vinyldimethicone Crosspolymer (Silikonelastomer KSP-100 der Shin Etsu Silicones of America Inc./USA) |
| 5,0 g | Cyclomethicone |
| 100,0 g | |

Zunächst werden die einzelnen Pulverbestandteile jeweils mit einer ausreichenden Menge einer Dispersion des Silkonelastomeren in dem Cyclomethicone behandelt. Die so erhaltenen mit dem Silikonelastomeren beschichteten Pulverbestandteile werden anschliessend in einem Mischer miteinander vermischt

### Beispiel 2: Staubfreie Blondierung

| | |
|---|---|
| 18,0 g | Ammoniumpersulfat |
| 23,0 g | Natriumperoxydisulfat |
| 23,0 g | Natriumsilicat, alkalisch |
| 22,0 g | Magnesiumcarbonat |
| 2,5 g | hochveretherte Methylhydroxyethylcellulose mit einer Viskosität von ca. 6000 mPas (Tylose MH 6000 YP2 der Fa. Clariant/DE) |
| 1,0 g | Ethylendiaminotetraacetat-Natriumsalz |
| 9,0 g | Cyclomethicone |
| 1,5 g | Dimethicone/Vinyldimethicone Crosspolymer (DOW CORNING 9506 der Dow Corning Corp./USA) |
| 100,0 g | |

Eine Dispersion des Dimethicone/Vinyldimethicone Crosspolymeren in dem Cyclomethicone wird mit den übrigen Pulverbestandteilen in einem Mischer vermischt.

### Beispiel 3: Pulverhaarfarbe wasseraktivierbar (Anwendung 1:4 mit Wasser)

| | |
|---|---|
| 30,0 g | Natriumpercarbonat |
| 20,0 g | hochveretherte Natriumcarboxymethylcellulose mit einer Viskosität von ca. 30000 mPas (Tylose CB 30000P der Fa. Clariant/DE) |
| 4,0 g | Natriumlaurylsulfat-Pulver |
| 10,0 g | Natriumsilikat, alkalisch |
| 1,5 g | Ethylendiaminoteraacetat-Natriumsalz |
| 0,5 g | Ascorbinsäure |
| 13,5 g | 1,4-Diaminobenzol x 2HCl |
| 5,0 g | 1,3-Diaminobenzol x 2HCl |
| 4,5 g | 2-Aminophenol |
| 1,0 g | Aluminiumstearat |
| 10,0 g | Cyclopentasiloxan (and) Dimethicone Crosspolymer (DOW CORNING 9040 der Dow Corning Corp./USA) |
| 100 g | |

Das Cyclopentasiloxan (and) Dimethicone Crosspolymer wird mit den übrigen Pulverbestandteilen in einem Mischer vermischt.

### Beispiel 4: Pflanzenhaarfarbe (Anwendung 1:1 bis 1:2 mit warmem Wasser)

| | |
|---|---|
| 90 g | Lawsonia Inermis |
| 2 g | Cyanopsis Tetragonalba |
| 3 g | Xanthan Gum |
| 5 g | Cyclopentasiloxan (and) Dimethicone Crosspolymer (DOW CORNING 9040 der Dow Corning Corp./USA) |
| 100 g | |

Das Cyclopentasiloxan (and) Dimethicone Crosspolymer wird mit den übrigen Pulverbestandteilen in einem Mischer vermischt.

### Beispiel 5: Pflanzenhaarfarbe Naturblond (Anwendung 1:1 bis 1:2 mit warmem Wasser)

| | |
|---|---|
| 40g | Cassia Auriculata |
| 25g | Acacia Catechu |
| 17g | Lawsonia Inermis |
| 6g | Salvia Officinalis |
| 2g | Cyanopsis Tetragonalba |
| 3g | Xanthan Gum |
| 7g | Cyclopentasiloxan (and) Dimethicone Crosspolymer (DOW CORNING 9040 der Dow Corning Corp./USA) |
| 100 g | |

Das Cyclopentasiloxan (and) Dimethicone Crosspolymer wird in einem Mischer langsam unter Rühren portionsweise den übrigen Pulverbestandteilen zugesetzt.

### Beispiel 6: Pulverhaarfarbe wasseraktivierbar (Anwendung 1:4 mit Wasser)

| | |
|---|---|
| 0,33 g | 2,5-Diamino-toluol-sulfat |
| 0,33 g | 2,5-Diamino-phenylethanol-sulfat |
| 0,22 g | N,N-Bis-(β-hydroxyethyl)-p-phenylendiamin-sulfat |
| 0,33 g | 4-Amino-3-methyl-phenol |
| 0,22 g | 2-Aminomethyl-p-aminophenol * HCl |
| 0,22 g | 4,5-Diamino-1-hydroxyethyl-pyrazol-sulfat |
| 0,22 g | 4,5-Diamino-1-(p-methylbenzyl)-pyrazol-sulfat |
| 0,11 g | 1-Naphtol |
| 0,11 g | 3,4-Diamino-benzoesäure |
| 0,11 g | 1-(β-Hydroxyethylamino)-3,4-methylendioxybenzol * HCl |
| 0,11 g | 2,4-Diamino-1-(β-hydroxyethoxy)benzol-sulfat |
| 0,11 g | 5-Amino-6-chlor-2-methyl-phenol |
| 0,11 g | 1,3-Bis-2,4-(diaminophenoxy)propan * 2HCl |
| 0,11 g | 3-Aminophenol |
| 0,11 g | 4-Chlor-resorcin |
| 0,11 g | 5-Amino-2-methyl-phenol |
| 0,11 g | 2-Amino-4-(β-hydroxyethylamino)-anisol-suffat |
| 0,11 g | 2,4-Diamino-1-fluor-5-methyl-benzol-sulfat (1:1) |
| 0,11 g | 3,5-Diamino-2,6-dimethoxy-pyridin * 2HCl |
| 0,11 g | Resorcin |
| 0,11 g | 2-Methyl-resorcin |
| 0,11 g | m-Dimethylaminophenylhamstoff |
| 0,02 g | 2-Amino-5-methyl-phenol |
| 0,02 g | 2-Amino-6-chlor-4-nitrophenol |
| 0,02 g | 4-(β-Hydroxyethylamino)-3-nitrophenol |
| 0,02 g | 4-[-3-Hydroxypropylamino]-3-nitro-phenol |
| 0,02 g | N1-(2-Hydroxyethyl)-2-nitro-p-phenylenediamin (HC Red 3) |
| 0,02 g | 4-Amino-3-nitrophenol |
| 0,02 g | 2-Amino-4,6-dinitrophenol |
| 0,02 g | 2-Hydroxyethyl-pikraminsäure |
| 0,02 g | 1-N-Hydroxyethylamino-4-methyl-2-nitrobenzol |
| 0,02 g | 6-Ethylamino-2-chlor-4-nitro-phenol |
| 0,02 g | 2,6-Diamino-3-((pyridin-3-yl)azo)pyridin |
| 0,02 g | 4-Nitrophenyl-aminoethylharnstoff |
| 30,00 g | Percarbonat |
| 20,00 g | hochveretherte Natriumcarboxymethylcellulose mit einer Viskosität von ca. 30000 mPas (Tylose CB 30000P der Fa. Clariant/DE) |
| 0,08 g | NaOH |
| 0,33 g | Ethylendiaminoteraacetat |
| 0,33 g | Na₂SO₃ |
| 1,5 g | Dimethicone/Vinyldimethicone Crosspolymer (DOW CORNING 9506 der Dow Corning Corp./USA) |

Das Dimethicone/Vinyldimethicone Crosspolymer wird mit den übrigen Pulverbestandteilen in einem Mischer vermischt.

Alle Prozentangaben stellen, soweit nicht anders angegeben, Gewichtsprozente dar.

## Patentansprüche

1. Pulverförmiges Blondiermittel auf der Basis einer festen Perverbindung, **dadurch gekennzeichnet, dass** es mindestens ein Silikonelastomer enthält, das ausgewählt ist aus Polymeren, welche durch Vernetzung von (I) Si-H-Gruppen enthaltenden Polysiloxanen mit (IIa) einem alpha, omega-Dien oder (IIb) einem Vinylpolysiloxan in Gegenwart eines Katalysators und eines Lösungsmittels erhalten werden.

2. Pulverförmiges Haarfärbemittel auf der Basis oxidativer und/oder nicht-oxidativer Farbstoffe, **dadurch gekennzeichnet, dass** es mindestens ein Silikonelastomer enthält, das ausgewählt ist aus Polymeren, welche durch Vernetzung von (I) Si-H-Gruppen enthaltenden polysiloxanen mit (IIa) einem alpha, omega-Dien oder (IIb) einem Vinylpolysiloxan in Gegenwart eines Katalysators und eines Lösungsmittels erhalten werden.

3. Mittel nach Anspruch 1 oder 2, **dadurch gekennzeichnet, dass** das Silikonelastomer ein Dimethicone/Vinyldimethicone Crosspolymer oder ein Cyclopentasiloxan (and) Dimethicone Crosspolymer ist.

4. Mittel nach einem der Ansprüche 1 bis 3, **dadurch gekennzeichnet, dass** das Silikonelastomer, bezogen auf die Gesamtmenge des Mittels, in einer Menge von 0,01 bis 20 Gewichtsprozent enthalten ist.

5. Verfahren zur Herstellung pulverförmiger Blondiermittel oder pulverförmiger Haarfärbemittel, bei dem ein die Bestandteile des Blondiermittels oder Haartärbemittels enthaltendes Pulver mit dem Silikonelastomeren das ausgewählt ist aus Polymeren, welche durch Vernetzung von (I) Si-H-Gruppen enthaltenden Polysiloxanen mit (IIa) einem alpha, omega-Dien oder (IIb) einem Vinylpolysiloxan in Gegenwart eines Katalysators und eines Lösungsmittels erhalten werden, in einem geeigneten Mischer bei Raumtemperatur behandelt wird.

6. Verfahren zur Herstellung pulverförmiger Blondiermittel oder Haarfärbemittel, bei dem zunächst die einzelnen Pulverbestandteile mit dem Silikonelastomeren das ausgewählt ist aus Polymeren, welche durch Vernetzung von (I) Si-H-Gruppen enthaltenden Polysiloxanen mit (IIa) einem alpha, omega-Dien oder (IIb) einem Vinylpolysiloxan in Gegenwart eines Katalysators und eines Lösungsmittels erhalten werden, in einem geeigneten Mischer bei Raumtemperatur behandelt und sodann miteinander vermischt werden.

7. Verfahren nach Anspruch 5 oder 6, **dadurch gekennzeichnet, dass** das Silikonelastomer ein Dimethicone/Vinyldimethicone Crosspolymer oder ein Cyclopentasiloxan (and) Dimethicone Crosspolymer ist.

8. Verfahren nach einem der Ansprüche 5 bis 7, **dadurch gekennzeichnet, dass** das die Bestandteile des Blondiermittels/Haarfärbemittels enthaltende Pulver in einem Mischer bei 10 bis 35 °C mit dem Silikonelastomer behandelt wird.

9. Verfahren nach einem der Ansprüche 5 bis 8, **dadurch gekennzeichnet, dass** das Silikonelastomer in einem Öl dispergiert ist.

10. Verwendung eines durch Vernetzung von (I) Si-H-Gruppen enthaltenden Polysiloxanen mit (IIa) einem alpha, omega-Dien oder (IIb) einem Vinylpolysiloxan in Gegenwart eines Katalysators und eines Lösungsmittels erhaltenen Silkonelastomeren zur Herstellung von staubfreien, lagerstabilen pulverförmigen Blondiermitteln/Haarfärbemitteln.

11. Verwendung nach Anspruch 10, **dadurch gekennzeichnet, dass** zur Herstellung der staubfreien, lagerstabilen pulverförmigen Blondiermittel/Haarfärbemittel ein Dimethicone/Vinyldimethicone Crosspolymer oder ein Cyclopentasiloxan (and) Dimethicone Crosspolymer verwendet wird.

## Claims

1. Bleaching composition in powder form based on a solid percompound, **characterized in that** it comprises at least one silicone elastomer which is chosen from polymers which are obtained by crosslinking (I) polysiloxanes containing Si-H groups with (IIa) an alpha, omega-diene or (IIb) a vinylpolysiloxane in the presence of a catalyst and a solvent.

2. Hair colorant in powder form based on oxidative and/or nonoxidative dyes, **characterized in that** it comprises at least one silicone elastomer which is chosen from polymers which are obtained by crosslinking (I) polysiloxanes containing Si-H groups with (IIa) an alpha, omega-diene or (IIb) a vinylpolysiloxane in the presence of a catalyst and a solvent.

3. Composition according to Claim 1 or 2, **characterized in that** the silicone elastomer is a dimethicone/vinyldimethicone crosspolymer or a cyclopentasiloxane (and) dimethicone crosspolymer.

4. Composition according to one of Claims 1 to 3, **characterized in that** the silicone elastomer, based on the total amount of the composition, is present in an amount of from 0.01 to 20 percent by weight.

5. Process for the preparation of bleaching compositions in powder form or hair colorants in powder form, in which a powder comprising the constituents of the bleaching composition or hair colorant is treated with the silicone elastomer which is chosen from polymers which are obtained by crosslinking (I) polysiloxanes containing Si-H groups with (IIa) an alpha, omega-diene or (IIb) a vinylpolysiloxane in the presence of a catalyst and a solvent, in a suitable mixer at room temperature.

6. Process for the preparation of bleaching compositions or hair colorants in powder form in which, firstly, the individual powder constituents are treated with the silicone elastomer which is chosen from polymers which are obtained by crosslinking (I) polysiloxanes containing Si-H groups with (IIa) an alpha, omega-diene or (IIb) a vinylpolysiloxane in the presence of a catalyst and a solvent, in a suitable mixer at room temperature, and then mixed together.

7. Process according to Claim 5 or 6, **characterized in that** the silicone elastomer is a dimethicone/vinyldimethicone crosspolymer or a cyclopentasiloxane (and) dimethicone crosspolymer.

8. Process according to one of Claims 5 to 7, **characterized in that** the powder comprising the constituents of the bleaching composition/hair colorant is treated with the silicone elastomer in a mixer at 10 to 35°C.

9. Process according to one of Claims 5 to 8, **characterized in that** the silicone elastomer is dispersed in an oil.

10. Use of a silicone elastomer obtained by crosslinking (I) polysiloxanes containing Si-H groups with (IIa) an alpha, omega-diene or (IIb) a vinylpolysiloxane in the presence of a catalyst and a solvent, for preparing dust-free, storage-stable bleaching compositions/hair colorants in powder form.

11. Use according to Claim 10, **characterized in that** a dimethicone/vinyldimethicone crosspolymer or a cyclopentasiloxane (and) dimethicone crosspolymer is used for preparing the dust-free, storage-stable bleaching compositions/hair colorants in powder form.

## Revendications

1. Composition pulvérulente pour la coloration en blond, à base d'un composé "per" solide, **caractérisée en ce qu'**elle contient au moins un élastomère silicone qui est choisi parmi des polymères qui sont obtenus par réticulation (I) de polysiloxanes contenant des groupes Si-H avec (IIa) un alpha,oméga-diène ou (IIb) un vinylpolysiloxane en présence d'un catalyseur et d'un solvant.

2. Composition pulvérulente de teinture pour cheveux, à base de colorants oxydants et/ou de colorants non oxydants, **caractérisée en ce qu'**elle contient au moins un élastomère silicone qui est choisi parmi des polymères qui sont obtenus par réticulation (I) de polysiloxanes contenant des groupes Si-H avec (IIa) un alpha,oméga-diène ou (IIb) un vinylpolysiloxane en présence d'un catalyseur et d'un solvant.

3. Composition selon la revendication 1 ou 2, **caractérisée en ce que** l'élastomère silicone est un polymère réticulé diméthicone/vinyldiméthicone ou un polymère réticulé cyclopentasiloxane (et) diméthicone.

4. Composition selon l'une quelconque des revendications 1 à 3, **caractérisée en ce que** l'élastomère silicone est contenu en une quantité de 0,01 à 20 % en poids, par rapport à la quantité totale de la composition.

5. Procédé pour la production de compositions pulvérulentes pour la coloration en blond ou de compositions pulvérulentes de teinture pour cheveux, dans lequel on traite dans un mélangeur approprié, à la température ambiante, une poudre contenant les composants de la composition de coloration en blond ou de la composition de teinture pour cheveux, par l'élastomère silicone qui est choisi parmi des polymères qui sont obtenus par réticulation (I) de polysiloxanes contenant des groupes Si-H avec (IIa) un alpha,oméga-diène ou (IIb) un vinylpolysiloxane en présence d'un catalyseur et d'un solvant.

6. Procédé pour la production de compositions pour la coloration en blond ou de compositions de tenture pour cheveux, pulvérulentes, dans lequel d'abord on traite les composants pulvérulents individuels dans un mélangeur approprié, à la température ambiante, par l'élastomère silicone qui est choisi parmi des polymères qui sont obtenus par réticulation (I) de polysiloxanes contenant des groupes Si-H avec (IIa) un alpha,oméga-diène ou (IIb) un vinylpolysiloxane en présence d'un catalyseur et d'un solvant, et ensuite on les mélange entre eux.

7. Procédé selon la revendication 5 ou 6, **caractérisée en ce que** l'élastomère silicone est un polymère réticulé diméthicone/vinyldiméthicone ou un polymère réticulé cyclopentasiloxane (et) diméthicone.

8. Procédé selon l'une quelconque des revendications 5 à 7, **caractérisé en ce que** la poudre contenant les composants de la composition pour coloration en blond/composition de teinture pour cheveux est traitée par l'élastomère silicone dans un mélangeur à 10-35 °C.

9. Procédé selon l'une quelconque des revendications 5 à 8, **caractérisé en ce que** l'élastomère silicone est dispersé dans une huile.

10. Utilisation d'un élastomère silicone obtenu par réticulation (I) de polysiloxanes contenant des groupes Si-H avec (IIa) un alpha,oméga-diène ou (IIb) un vinylpolysiloxane en présence d'un catalyseur et d'un solvant, pour la production de compositions pour la coloration en blond/compositions de teinture pour cheveux, pulvérulentes, stables au stockage, dépourvues de poussière.

11. Utilisation selon la revendication 10, **caractérisée en ce que** pour la production de compositions pour la coloration en blond/compositions de teinture pour cheveux, pulvérulentes, stables au stockage, dépourvues de poussière, on utilise un polymère réticulé diméthicone/vinyldiméthicone ou un polymère réticulé cyclopentasiloxane (et) diméthicone.
